# EUROPEAN PATENT SPECIFICATION

(11) **EP 4 107 303 B1**
(45) Date of publication and mention of the grant of the patent: **06.09.2023**
(21) Application number: 21705531.8
(22) Date of filing: 17.02.2021
(51) Int. Cl.: C22C 30/00, A61F 2/82, C22C 30/02, C22C 30/06, C22C 38/00, C22C 38/04, C22C 38/06, C22C 38/16

(54) **USE OF BIORESORBABLE PSEUDOELASTIC FE-MN-X-Y ALLOYS FOR MEDICAL IMPLANTS**
VERWENDUNG VON BIORESORBIERBAREN PSEUDOELASTISCHEN FE-MN-X-Y-LEGIERUNGEN FÜR MEDIZINISCHE IMPLANTATE
UTILISATIONS D'ALLIAGES FE-MN-X-Y PSEUDO-ÉLASTIQUES BIORÉSORBABLES POUR IMPLANTS MÉDICAUX

(30) Priority: 21.02.2020 IT 202000003611
(43) Date of publication of application: 28.12.2022
(73) Proprietor: Saes Getters S.p.A., 20020 Lainate (MI) (IT)
(72) Inventor: CODA, Alberto, 21040 Cislago VA (IT); LEMKE, Jannis, 20148 Milano MI (IT); TUISSI, Ausonio, 23900 Lecco LC (IT); FIOCCHI, Jacopo, 23900 Lecco LC (IT)
(74) Representative: Concone, Emanuele
(86) International application number: PCT/EP2021/053909
(87) International publication number: WO 2021/165333

(56) References cited:
- TW-A- 201 313 923
- KIM MIN-SU ET AL: "Development of thermodynamic database for high Mn-high Al steels: Phase equilibria in the Fe-Mn-Al-C system by experiment and thermodynamic modeling", CALPHAD. COMPUTER COUPLING OF PHASE DIAGRAMS AND THERMOCHEMISTRY, vol. 51, 4 September 2015 (2015-09-04), pages 89-103, XP055793918, US ISSN: 0364-5916, DOI: 10.1016/j.calphad.2015.08.004
- ZAMBRANO O A ED - GLADYSZ G M ET AL: "A general perspective of Fe-Mn-Al-C steels", JOURNAL OF MATERIAL SCIENCE, KLUWER ACADEMIC PUBLISHERS, DORDRECHT, vol. 53, no. 20, 20 June 2018 (2018-06-20) , pages 14003-14062, XP036559866, ISSN: 0022-2461, DOI: 10.1007/S10853-018-2551-6 [retrieved on 2018-06-20]
- APERADOR W ET AL: "BIOMATERIALS BASED IN FERMANAL STEEL, AN OPTION TO THE SURGICAL IMPLANTS MANUFACTURING", REVISTA MEXICANA DE INGENIERÍA QUÍMICA, vol. 12, no. 2, 31 August 2013 (2013-08-31), pages 337-344, XP055793921,

## Description

The present invention relates to metal alloy compositions with pseudoelastic properties, exhibiting an adequate biodegradation rate with excellent mechanical properties, suitable to be used for manufacturing bioresorbable medical implants.

The function of said implants would be as a structure (or scaffold) to support tissue growth, to replace bone for fracture fixation, as vascular stents to keep blood vessels open and in several other medical applications. An important aspect regards the duration of the healing process, that depends on the patient conditions, e.g. extent of the injury, age, further medical treatments, etc. The use of permanent implants requires their removal after the completion of the healing process to avoid harmful side effects in the long term. These issues are even more relevant in pediatrics, where the rapid growth development of young patients makes the use of permanent implants unsuitable.

Bioresorbable implants are designed to degrade and disappear in an application-relevant time scale, they do not need to be removed after being inserted into the body to achieve their desired purpose.

As used herein, "biocorrodible," "bioabsorbable" and "bioresorbable" all refer to a material that can be dissolved and absorbed into a living tissue, and thus does not need to be removed by a second surgery on a time scale which can vary from a few months to several years, depending on its biological environment (e.g. health condition, type of implant, patient's conditions and response to the treatment). The bioresorbable mechanism is to be understood as a set of hydrolytic, enzymatic and metabolic processes in the body, in which the physiologic fluids get in contact with the biomaterial producing oxides, hydroxides and other compounds, the result of these chemical reactions leading to a gradual degradation of the implant structure. To date, several alloys have been investigated to obtain a bioresorbable alloy and among them the three most investigated classes are represented by iron-based alloys, zinc-based alloys and magnesium-based alloys.

Superior mechanical properties of metals, compared to other materials in terms of obtaining high deformations combined with high strength, make possible the manufacture of implants with thin structures that can bear rather high loads as needed in many vascular applications, but also in several orthopedic applications, to replace hard tissue.

Generally, high elastic modulus (higher than 100 GPa), high yield strength (higher than 300 MPa) and a good ductility (above 18% deformation prior to failure) are targeted in the development of vascular implant materials. Moreover, good ductility is also considered as an important characteristic in the processing from base material to implant with a desired shape. The most used metals for biomedical applications and implants such as stainless steel 316L show moderate yield strength (i.e. comprised in the 150-250 MPa) and adequate elastic modulus (~190 GPa), while pure Fe components show a yield strength of about 150 MPa and an elastic modulus of ~200GPa in annealed conditions.

Another alloy widely applied in the medical sector due to its excellent mechanical properties is Ni-Ti. If mechanically loaded, pseudoelastic Ni-Ti-based alloys might deform reversibly up to very high strains (up to 10%) by the phase transformation from austenite to stress-induced Martensite and when the load is removed the new phase becomes unstable and the material recovers its original shape by back transformation. The effect is correlated to the common shape memory effect in Ni-Ti alloys. But unlike shape-memory alloys, no change in temperature is needed for the alloy to recover its initial shape when the material is in its austenitic state. The term "superelasticity" is often used alternatively for "pseudoelasticity" and both have been used to describe this transformation-induced nonlinear elasticity. A general review on shape memory and superelastic alloys can be found in "Shape memory alloys behavior: A review", by Lobo et al. in Procedia Engineering 114 (2015) 776-783 (2015) and in "A review of shape memory alloy research, applications and opportunities" by Jani et al. in Materials and Design 56:1078-1113 (2014).

Superelastic devices take advantage of their large, reversible deformation and among the most important applications there are medical stents.

Other important aspects in selecting a metallic material for biomedical applications are its biocompatibility and corrosion behavior (which has a significant impact on the biocompatibility) in the biological environment of application. From this perspective, an adequate corrosion speed can be turned into a benefit when it comes to bioresorbable metallic implants.

Iron-based alloys usually have attractive mechanical properties, which make them suitable for coronary implants, but their corrosion rate is very slow, with possible long-term side effects. Several studies have conclusively suggested that bioresorbable vascular Fe scaffolds substantially dissolve over time, usually within a period of few years.

On the contrary, zinc alloys, which are also considered for biodegradable vascular and bones implants, can corrode faster than Fe alloys, but there is controversy on the allowed daily amount of Zn to which the human body can be exposed without health risks. Furthermore, the mechanical properties of Zn alloys, even though they can be improved to some extent by alloying elements, remain inferior to iron-based alloys.

Magnesium implants appear highly biocompatible in biological environments. In orthopedic applications selected magnesium alloys would reduce implant-associated stress shielding and bone degradation, for example magnesium-based screws have been used with good results in bone healing clinical trials.

The main drawbacks of magnesium and its alloys are their relatively low strength and ductility, the release of hydrogen for chemical reaction with water and water-based solutions, the high corrosion rate, the latter considered particularly limiting for implant applications which have to bear mechanical loads, as failure could occur before the healing process is completed and thick structures are needed to compensate for low strength.

Among the several solutions that have been disclosed in order to provide bioresorbable alloys for medical implants, US 8372144 describes a base body for an implant comprising a biocorrodible Fe-Mn-X alloy where Mn is 5-30 wt% of the alloy composition, and X is selected among the noble elements Pt, Pd, Ir, Rh, Re, Ru and Os, comprised between 0 and 20 wt%. The purpose of adding a third alloying element is to reduce the corrosion resistance of binary iron-manganese alloys.

US 20170360998 discloses a bioabsorbable wire material including manganese (Mn) and iron (Fe), in which one or more additional elements are added to control corrosion in an in vivo environment and, in particular, to prevent and/or substantially reduce the potential for pitting corrosion. In this case, the additional element in the Fe-Mn-X system has been selected among nitrogen (N), molybdenum (Mo) or chromium (Cr).

WO 2017188908 describes a tubular metal alloy stent, wherein a Fe-Mn-X-Y alloy is used for producing biodegradable stents. Fe is characterized as 47-75% by weight, Mn is 20-35% by weight, X contains at least one element selected from the group that is composed of Si, Co, Mo and Y contains at least one element selected from the group that is composed of C and Pd.

CN 104694848 describes biodegradable Fe-Mn-X-Y alloys wherein X includes one of Mg, Zn and Ag in an amount of 1-3 wt% and Y includes one of C, Si and S in an amount of 0.5-3 wt%, where the content of Mn is always disclosed in an amount equal to 30 wt%.

It is indeed important to point out that several prior art documents, such as EP 1555332 and the paper "Development of the novel ferrous-based stainless steel for biomedical applications, Part I: high-temperature microstructure, mechanical properties and damping behavior", Journal of the Mechanical Behavior of Biomedical Materials, Elsevier, Amsterdam, NL, Vol.4, No.7; pp.1548-53; 2011.02.10, disclose Fe-Mn-Al-C alloys defined biocompatible which, contrary to our invention, must not degrade in the human body but have an excellent corrosion resistance to remain unaltered, with a consequent corrosion rate significantly different from that developed by our inventors.

Other examples can be found in the paper "Development of thermodynamic database for high Mn-high Al steels: Phase equilibria in the Fe-Mn-Al-C system by experiment and thermodynamic modeling", CALPHAD. COMPUTER COUPLING OF PHASE DIAGRAMS AND THERMOCHEMISTRY, 51, 2015.09.04, pp.89-103, XP055793918 in which the Fe-Mn-Al-C alloys are studied for applications in fields such as automotive, cryogenic, electrical steels, etc. as well as in the paper "A general perspective of Fe-Mn-Al-C steels", J Mater Sci, 2018.06.20, Vol. 53, No. 20, pp.14003-14062, XP036559866 which mentions the possible biocompatibility of these alloys without any indication or hint to them being bioresorbable.

Iron-based solutions of the prior art have several limits, consisting in some cases in alloys having poor mechanical properties with limited elongation to failure or no pseudoelasticity, in some others in alloys with limited pseudoelasticity which however contain elements like Ni or Co that are considered in this field elements with poor or no biocompatibility.

The invention is disclosed in the appended claims. Purpose of this invention is to disclose new iron-manganese based alloys that show suitable degradation characteristics and mechanical properties at the same time, in particular for some types of medical implants as for example thin-walled vascular implants characterized by a thickness comprised between 20 and 300 µm, typically applied to implants with a diameter between 1 and 5 mm and a length comprised between 5 and 50 mm.

In particular, the purpose of the present invention is to improve the performance of biodegradable alloys of the prior art for implant applications which are exposed to significant mechanical loads or can benefit from miniaturization. The present alloys offer particular mechanical properties as to pseudoelasticity, high product of strength and ductility (also referred to by the ECO-index: ultimate tensile strength × elongation to rupture) with sufficiently high corrosion rates. The combination of these properties allows for engineering thin implant devices which degrade in application-required time periods.

Furthermore, pseudoelasticity, known in particular from non-degradable NiTi alloys in the biomedical sector, is a highly appreciated property to avoid permanent implant damage during deformation and to facilitate implanting. In medical devices, in particular thin-walled stents, the pseudoelastic effect is important to allow in-service large recoverable deformation of the device.

The Fe-Mn-X-Y alloys disclosed herein contain iron and manganese as main components. Iron, as described above, if considered as single element, has a degradation rate in most biological environments found to be excessively slow to be practical for bioresorbable applications. The alloying of manganese reveals several advantages: it is a nutrient element and thus considered biocompatible, it improves mechanical properties and constitutes an approach to increase iron's corrosion rate by reducing its corrosion potential. Furthermore, Mn is a GAMMA-iron (austenite) stabilizer such that by adding high quantities of Mn the ferromagnetic ALPHA-iron phase becomes less stable and the material becomes antiferromagnetic or paramagnetic. Avoiding ferromagnetic behavior is considered important to guarantee compatibility with MRI scans.

The manganese content has an important impact on the deformation mechanisms in Fe-Mn. Different mechanisms are described in literature, among those are transformation-induced plasticity (TRIP), twinning-induced plasticity (TWIP), microband-induced plasticity (MIP), slip band refinement plasticity (SRIP) which allow for an excellent ductility despite being paired with a high strength. In this regard, the role of the above effect in achieving high strength and ductility is strongly considered for the invention purposes.

Fe-Mn-based alloys disclosed herein are characterized by the presence of a transforming matrix phase at room temperature which either can be GAMMA- or ALPHA-phase in an either monophasic or bi-phasic microstructure. While the microstructure may contain more than one microstructural phase, the pseudoelastic response correlates to the amount of transformable matrix.

However, in order to exhibit pseudoelastic behavior, solely a transformable matrix is not sufficient, but also the presence of nano-scale coherent precipitates is required to render a back transformation after unloading possible. The presence of coherent precipitates in the matrix, is important to avoid an extensive amount of precipitates at grain boundaries which can cause embrittlement of the alloys. Furthermore, the presence of precipitates in adequate amount and size can be exploited to accelerate/decelerate the corrosion rate. For this scope, the elevated amount of Mn in the alloys supports further tailoring of the corrosion rate by controlled formation and dissolution of β-Mn upon using thermomechanical treatments.

The bioresorbable metal alloy-containing composition Fe-Mn-X-Y herein disclosed further comprises, as additional compositional elements, X and Y acting in their main role as precipitate-former elements and in addition at least one of them has an important role in stabilizing either GAMMA- or ALPHA-phase so as to obtain a high amount of transforming matrix Said additional element X is selected in the group consisting of at least one among aluminium, zinc, gallium and combinations thereof, while the precipitate former element Y is selected among carbon, copper and combinations thereof.

Metallic base materials contain unavoidable impurities, among those O, N, C and S are typically present in the metals used for the described alloys. The amount of such impurities should not exceed 0.5 at% as detrimental secondary phases could form.

It has been found that the right ratio of Fe:Mn:X:Y leads to pseudoelastic recovery after mechanically deforming the alloy.

In a first embodiment Fe and Mn atomic percentages are respectively comprised between 35.0 and 50.5 at%, and between 34.0 and 40.0 at%, wherein the amount of Fe is higher than the amount of Mn. Depending on the selection of alloying elements X and Y, a wide range of elemental ratios exists leading to the appearance of coherent precipitates which are fundamental for achieving pseudoelasticity. Specifically, the total amount of the sum of X and Y is in a range comprised between 12.5 and 26.0 at% of the alloy composition and the atomic ratio between X and Y is comprised between 1.0 and 3.6 to achieve a minimum pseudoelasticity, each alloy composition will have a specific much narrower range to maximize the pseudoelastic response. In specific embodiments said atomic ratio between X and Y elements is preferably comprised between 1.5 and 3.2, and the sum of X and Y between 13.0 and 25.0 at%.

According to a preferred embodiment, said bioresorbable metal alloy is characterized by the Fe content comprised between 43.0 and 50.5 at%, and the Mn content comprised between 34.0 and 37.5 at%.

In a further embodiment the content of X element is comprised between 10.0 and 15.0 at%, and the content of Y element is comprised between 5.0 and 8.0 at%.

Best results for a Fe-Mn-Al-Y alloy were obtained when aluminium is present in an amount comprised between 8.0 and 16.0 at%, and Y is carbon or copper in an amount comprised between 4.5 and 10.0 at%.

Moreover, for the disclosed medical implants application, it has been found to be particularly interesting the bioresorbable metal alloy composition containing Zn as X element (i.e. Fe-Mn-Zn-Y) with a preferable amount of Zn comprised between 8.0. at% and 16.0 at% and a carbon or copper (Y element) amount comprised between 4.5 and 10.0 at%.

The above defined ratios and amounts of elements in atomic percentages of these alloys can be engineered by thermal/thermo-mechanical treatments to modify the microstructural features, including reinforcing particle precipitation, formation of β-Mn, the matrix phase at room temperature and the herewith related phase transformations. The presence of precipitates due to the addition of elements X and Y in different combinations to iron and manganese not only affects pseudoelasticity, but also affects all mechanical properties and especially improves yield and tensile strength. Furthermore, the potential to induce coherent precipitates can improve not only pseudoelasticity and strength, but also the wear properties of the alloy.

It was found that these alloys showed excellent values regarding recovery upon deformation, elongation up to failure, high elastic modulus, high yield strength, ultimate tensile strength, compared to mechanical properties of reference materials for implants. Targeted mechanical properties for cardiovascular applications are >100 GPa for elastic modulus, >200 MPa for yield strength and >300 MPa for ultimate tensile strength. In this regard, the Fe-Mn-based alloys disclosed herein provide excellent values. Best results are achieved with Fe-Mn alloying selecting Al as X element and Cu or C as Y element.

A further advantage is related to obtaining an adequate degradation rate through high Mn content and the alloying of appropriate percentages and ratios of the X-Y elements and through their combination in the alloys. When considering the degradation rates, they should be slow enough to guarantee the mechanical integrity of the implant over the healing process, and fast enough to ensure that it would not cause any damage related to an extended stay in the body. A medical implant fabricated in accordance with the alloy compositions described herein will degrade and preferably dissolve completely within an adequate time frame.

Corrosion tests of Fe-Mn-X-Y alloys showed them to have high corrosion rates compared to pure iron as well as several other Fe-Mn-based alloys, containing an equivalent amount of element X and Mn in the range of 20.0 - 33.0 at% disclosed in the prior art.

These Fe-Mn-X-Y alloys show in corrosion test performed according to ASTM G5 - 94 R99 corrosion rates intermediate between pure Fe and Mn alloys. In addition, account must be taken of the fact that the high mechanical properties of the alloys allow for engineering so small structures that the final device might be bioabsorbed in a significantly shorter time than devices of materials with equivalent or even higher corrosion rates, but higher thickness.

These Fe-Mn-X-Y alloys can be processed into various absorbable medical implants, such as peripheral vascular stents, coronary vascular stents, neurovascular stents, tracheal stents, bile duct stents and esophagus stents or implants in cavities and tracts, with particular reference to thin wall medical implants.

The alloys for bioresorbable medical implants disclosed herein can be produced by melting the pure elements, preferably in powder or pieces, in order to obtain the desired atomic ratios and concentrations. The melting must be carried out in a controlled atmosphere, for example under vacuum or inert gas, in order to avoid the oxidation of the alloy which is being prepared and/or to keep under control the high vapor pressure of some elements, such as Mn, Zn, or Cu to cite the most relevant. The most common melting technologies that can be used, but not limited thereto, are arc melting, vacuum induction melting (VIM), vacuum arc remelting (VAR), induction skull melting (ISM), electro slug remelting (ESR), or electron beam melting (EBM).

Further, a set of processing steps executed are related to heat treatments. In its broadest sense, the heat treatment refers to any of the heating and cooling operations that are performed for the purpose of changing the mechanical properties, the microstructure or the residual stress state of a metal product.

For example, solution treatment in combination with quenching are performed to solubilize secondary phases and to freeze the material in a desired microstructural state, e.g. a certain quantity of ALPHA/GAMMA-phase that forms at higher temperatures. Homogenization/normalizing is used prior to hot working processes and is performed to obtain a homogenous microstructure of medium grain size. Precipitation treatments are preformed to induce homogenously coherent precipitates in a prior solution-treated material. Stress release treatments are applied to relax the material without causing abundant precipitation.

A cycling treatment (heating in cycles an alloy between two high temperatures) can be performed, in the specific case for biphasic alloys, to induce "abnormal grain growth". The material is heated for 2-10 cycles between 850 - 1250°C for time spans of 5 - 60 min.

A further set of processing steps executed are related to main deformation processes and the ways in which they are used to form metallic objects of various shape or geometry. Forming processes include stamping, rolling, extrusion and forging, where deformation is induced by external compressive forces or stresses exceeding the yield stress of the material. Forming can be divided principally into two categories: hot working and cold working. At hot working temperatures, a metal remains ductile through dynamic reforming of its grain structure, so that repeated, large deformations are possible. The strain rates of many metal-working processes are so high that there is insufficient time for the metal to recrystallize as it deforms.

Cold working is inherent to processes that are performed at room temperature (or up to about 200°C for some metals), leads to anisotropy and increased stiffness and strength in a metal. There is a corresponding decrease in ductility and malleability as the metal strain hardens. Advantages over hot working include a better quality surface finish, closer dimensional control of the final article and improved mechanical properties.

By the above mentioned forming and processing techniques, thin semi-finished products like wire, sheets and tubes made out of the invented alloys could be prepared. Subsequently, these alloys are converted into a medical devices using precision cutting methods and surface refinement treatments like electro-polishing. Furthermore, post processing methods could be necessary to prepare the device for final use, e.g. deposition of a drug-releasing coating in case of a cardiovascular-stent implant.

The invention will be further illustrated by means of the following examples. These non-limiting examples are intended to teach the skilled person how to put the invention into practice.

### EXAMPLE 1

Experimental tests were carried out to provide information about the mechanical properties of bioresorbable Fe-Mn-based metal alloys. Samples S1, S2, S3, S4, S5, S6, S7, S8, S9 represent alloy compositions according to the claims and tested in two conditions, namely as homogenized ingot (HOMO) and after homogenizing and cold rolling (CR) with heat treatment at 500°C for 6 hours.

Some comparative samples C1, C2, C3, C4, C9, C10, C11 represent general mechanical properties for the most common cardiovascular material.

C1 is 316L, a stainless steel, more specifically an austenitic chromium-nickel stainless steel that contains between 2 and 3 at% molybdenum.

C2 and C3 represent pure Fe and Mg, C4 represent a high strength Mg-based alloy WE43. The chemical composition of C4 is mainly characterized by magnesium and small percentage of Yttrium (3.7-4.3 at%), rare earths (2.4-4.4 at%) and zirconium (0.4 at%). C9, C10 and C11 consist of alloys of the same elements as S1-S9, but out of the claimed compositional ranges. These alloys were excessively brittle to be manufactured or tested, in nothing comparable to the excellent mechanical properties obtained in samples S1 - S9.

The tests concerning the measures of elongation, elastic modulus, yield strength, ultimate tensile strength in order to evaluate mechanical properties of the alloys are summarized in table A. The results reveal that Fe-Mn alloys with addition of Al, C, and Cu have values demonstrating excellent mechanical properties in relation to comparative samples. In fact it can be seen that elongation, elastic modulus (E), yield strength (YS) and ultimate tensile strength (UTS) of S1, S2, S3, S4, S5, S6, S7, S8, S9 tested in the above-mentioned two conditions reach values significantly higher than comparative samples C1, C2, C3, C4, especially compared to pure Fe and WE43.

**Table A**

| | Alloy | Composition [at.%] | | | | Condition | Elongation (%) | E (GPa) | YS (MPa) | UTS (MPa) |
|---|---|---|---|---|---|---|---|---|---|---|
| | | Fe | Mn | Al | Y | | | | | |
| S1 | FeMnAIC | 44.6 | 35.0 | 15.0 | 4.9 | HOMO Ingot | 53 | 210 | 350 | 675 |
| S2 | FeMnAIC | 44.6 | 35.0 | 15.0 | 4.9 | CR+500°C × 6h | 28 | 160 | 650 | 912 |
| S3 | FeMnAICu | 43.5 | 34.0 | 15.0 | 7.5 | HOMO Ingot | 33 | 186 | 480 | 741 |
| S4 | FeMnAICu | 43.5 | 34.0 | 15.0 | 7.5 | CR+500°C × 6h | 9 | 233 | 690 | 748 |
| S5 | FeMnAIC | 50.5 | 36.5 | 8.0 | 5.0. | HOMO Ingot | 43 | 135 | 408 | 801 |
| S6 | FeMnAlC | 50.5 | 36.5 | 8.0 | 5.0 | CR+500°C × 6h | 11 | 169 | 928 | 1035 |
| S7 | FeMnAICu | 44.6 | 35.4 | 15.0 | 5.0 | HOMO Ingot | 31 | 150 | 260 | 589 |
| S8 | FeMnAlCu | 44.6 | 35.4 | 15.0 | 5.0 | CR+500°C × 6h | 8 | 138 | 305 | 524 |
| S9 | FeMnAIC | 47.6 | 37.4 | 10.0 | 5.0 | HOMO Ingot | 40 | 157 | 741 | 970 |
| C1 | 316L | n.a. | n.a. | n.a. | n.a. | n.a. | 40 | 193 | 190 | 490 |
| C2 | Pure Fe | n.a. | n.a. | n.a. | n.a. | As cast | 40 | 200 | 150 | 200 |
| C3 | Pure Mg | n.a. | n.a. | n.a. | n.a. | Extruded | 13 | 45 | 20 | 86 |
| C4 | WE43 | n.a. | n.a. | n.a. | n.a. | n.a. | 2 | 44 | 170 | 220 |
| C9 | FeMnAICu | 44.0 | 33.0 | 12.0 | 11.0 | Brittle, ruptured during rolling | | | | |
| C10 | FeMnAIC | 35.0 | 32.0 | 15.0 | 18.0 | Brittle, ruptured during rolling | | | | |
| C11 | FeMnAl | 46.9 | 37.1 | 16.0 | - | Brittle, ruptured during rolling | | | | |

### EXAMPLE 2

The corrosion properties have been evaluated by potentiostatic and potentiodynamic tests in presence of oxygen according to ASTM G 5 - 94 R99 in order to measure relevant corrosion parameters. like the corrosion potential (Ecorr) and corrosion rate (mm/y). Samples C5, C6, C7 represent FeMn binary alloys, C8 a ternary alloy with the addition of Al, C12 a FeMnAlC out of the claimed ranges and samples S1, S3, S5, S7, S9 represent FeMnAlC and FeMnAlCu alloys in homogenized state. Comparative samples C2 and C3 represent pure Fe and Mg respectively.

Results have been summarized in Table B that shows how the corrosion potential and corrosion rate values demonstrate a good metal corrosion compared to pure Fe and pure Mg.

**Table B**

| Sample | Alloy | Fe (at%) | Mn (at%) | Al (at%) | Y (at%) | Condition | Ecorr (V) | Corrosion Rate (mm/y) |
|---|---|---|---|---|---|---|---|---|
| S1 | FcMnAIC | 44.6 | 35.0 | 15.0 | 4.9 | Homo | -0.55 | 0.04 |
| S3 | FeMnAICu | 43.5 | 34.0 | 15.0 | 7.5 | Homo | -0.64 | 0.05 |
| S5 | FeMnAIC | 50.5 | 36.5 | 8.0 | 5.0 | Homo | -0.67 | 0.06 |
| S7 | FeMnAICu | 44.6 | 35.4 | 15.0 | 5.0 | Homo | -0.65 | 0.03 |
| S9 | FeMnAIC | 47.6 | 37.4 | 10.0 | 5.0 | Homo | -0.65 | 0.21 |
| C2 | Pure Fe | n.a | n.a | n.a | n.a | As cast | -0.38 | 0.01 |
| C3 | Pure Mg | n.a | n.a | n.a | n.a | Extruded | -1.85 | 1.94 |
| C5 | Fe-Mn | 75.5 | 24.5 | n.a | n.a | Homo | -0.87 | 0.38 |
| C6 | Fe-Mn | 70.5 | 29.5 | n.a | n.a | Homo | -0.87 | 0.24 |
| C7 | Fe-Mn | 64.5 | 33.5 | n.a | n.a | Homo | -0.87 | 0.20 |
| C8 | FeMnAl | 57.3 | 26.2 | 16.6 | n.a | Homo | -0.74 | 0.02 |
| C12 | FeMnAlC | 48.0 | 30.0 | 16.6 | 5.4 | Homo | -0.59 | 0.01 |

### EXAMPLE 3

Dog bone samples with a gauge length of 20 ± 0.05 mm, a width of 1.5 ± 0.05 mm and a thickness of 1 ± 0.05 mm were mounted in a tensile testing device for investigating on pseudo-elastic properties. Therefore, samples were deformed in increments of 1% up to 8%, measuring the amount of recovery upon unloading after each step. The amount of recovery upon deforming to 8% is shown in table C.

Recovery values (%) are expressed as the percent amount of recovery with respect to the applied maximum deformation. Results show excellent pseudo-elastic properties tested in two conditions, which results in large recoverable deformation of the alloys S1, S2, S3, S4, S5, S10 particularly suitable for the purpose of the invention, final devices and applications. C13 is a FeMnAlCu alloy out of the claimed ranges, showing a low recovery below 10%.

**Table C**

| Sample | Alloy | Composition [at.%] | | | | Condition | Recovery (%) |
|---|---|---|---|---|---|---|---|
| | | Fe | Mn | Al | Y | | |
| S1 | FeMnAIC | 44.6 | 35.0 | 15.0 | 4.9 | HOMO Ingot | 19% |
| S2 | FeMnAIC | 44.6 | 35.0 | 15.0 | 4.9 | CR+500°C × 6h | 53% |
| S3 | FeMnAICu | 43.5 | 34.0 | 15.0 | 7.5 | HOMO Ingot | 23% |
| S4 | FeMnAICu | 43.5 | 34.0 | 15.0 | 7.5 | CR+500°C × 6h | 29% |
| S5 | FeMnAIC | 50.5 | 36.5 | 8.0 | 5.0 | HOMO Ingot | 10% |
| S10 | FeMnAIC | 43.4 | 34.1 | 15.0 | 7.5 | CR+500°C × 6h | 12% |
| C13 | FeMnAlCu | 48.5 | 35.0 | 15.0 | 1.5 | CR+500°C × 6h | 4% |

## Claims

1. Use in a vascular medical implant of a bioresorbable metal alloy composition consisting of Fe-Mn-X-Y plus unavoidable impurities that do not exceed 0.5 at% and wherein the amount of Fe is higher than the amount of Mn, **characterized in that** the Fe content is in an amount comprised between 35.0 and 50.5 at%, the Mn content is in an amount comprised between 34.0 and 40.0 at%, X is at least an element selected in the group consisting of aluminium, zinc, gallium and combinations thereof in an amount comprised between 8.0 and 16.0 at%, Y is copper or carbon or a combination thereof in an amount comprised between 4.5 and 10.0 at%, the atomic ratio between X and Y is comprised between 1.0 and 3.6 and the sum of X and Y is comprised between 12.5 and 26.0 at%.

2. Use in a vascular medical implant of the bioresorbable metal alloy composition according to claim 1, wherein the Fe content is comprised between 43.0 and 50.5 at%.

3. Use in a vascular medical implant of the bioresorbable metal alloy composition according to any of the previous claims, wherein the Mn content is comprised between 34.0 and 37.5 at%.

4. Use in a vascular medical implant of the bioresorbable metal alloy composition according to any of the previous claims, wherein the X element content is comprised between 10.0 and 15.0 at%.

5. Use in a vascular medical implant of the bioresorbable metal alloy composition according to any of the previous claims, wherein the Y element content is comprised between 5.0 and 8.0 at%.

6. Use in a vascular medical implant of the bioresorbable metal alloy composition according to any of the previous claims, wherein the sum of X and Y is comprised between 13.0 and 25.0 at%.

7. Use in a vascular medical implant of the bioresorbable metal alloy composition according to any of the previous claims, wherein the ratio of X and Y is comprised between 1.5 and 3.2.

8. Use in a vascular medical implant of the bioresorbable metal alloy composition according to claim 1 wherein the X element is aluminium.

9. Use in a vascular medical implant of the bioresorbable metal alloy composition according to any of claims 1 to 7, wherein the X element is zinc.

10. Use in a vascular medical implant of the bioresorbable metal alloy composition according to any of the previous claims, wherein the vascular medical implant is selected in a group consisting of peripheral vascular stents, coronary vascular stents, neurovascular stents, tracheal stents, bile duct stents and esophagus stents or implants in cavities and tracts.

11. A bioresorbable vascular medical implant comprising a bioresorbable metal alloy composition consisting of Fe-Mn-X-Y plus unavoidable impurities that do not exceed 0.5 at% and wherein the amount of Fe is higher than the amount of Mn, the Fe content is in an amount comprised between 35.0 and 50.5 at%, the Mn content is in an amount comprised between 34.0 and 40.0 at%, X is at least an element selected in the group consisting of aluminium, zinc, gallium and combinations thereof in an amount comprised between 8.0 and 16.0 at%, and Y is copper or carbon or a combination thereof in an amount comprised between 4.5 and 10.0 at%, wherein the atomic ratio between X and Y is comprised between 1.0 and 3.6, and the sum of X and Y is comprised between 12.5 and 26.0 at%.

12. A bioresorbable vascular medical implant according to claim 11, wherein the Fe content is in an amount comprised between 43.0 and 50.5 at%, the Mn content is in an amount comprised between 34.0 and 37.5 at%, X is in an amount comprised between 10.0 and 15.0 at% and Y is in an amount comprised between 5.0 and 8.0 at%, wherein the atomic ratio between X and Y is comprised between 1.5 and 3.2 and the sum of X and Y is comprised between 13.0 and 25.0 at%.

13. A bioresorbable vascular medical implant according to claim 11 or 12, selected in a group consisting of peripheral vascular stents, coronary vascular stents, neurovascular stents, tracheal stents, bile duct stents and esophagus stents or implants in cavities and tracts.

14. A bioresorbable vascular medical implant according to claim 11 or 12, wherein said medical implant is a thin-walled medical implant.

## Patentansprüche

1. Verwendung einer bioresorbierbaren Metalllegierungszusammensetzung bestehend aus Fe-Mn-X-Y plus unvermeidbaren Verunreinigungen, die 0,5 At.-% nicht überschreiten, und wobei der Fe-Gehalt höher ist als der Mn-Gehalt, in einem vaskulären medizinischen Implantat, **dadurch gekennzeichnet, dass** der Fe-Gehalt in einer Menge zwischen 35,0 und 50,5 At.-% liegt, der Mn-Gehalt in einer Menge zwischen 34,0 und 40,0 At.-% liegt, X mindestens ein Element ausgewählt aus der Gruppe bestehend aus Aluminium, Zink, Gallium und Kombinationen davon in einer Menge zwischen 8,0 und 16,0 At.-% ist, und Y Kupfer oder Kohlenstoff oder eine Kombination davon in einer Menge zwischen 4,5 und 10,0 At.-% ist, das atomare Verhältnis zwischen X und Y zwischen 1,0 und 3,6 liegt, und die Summe von X und Y zwischen 12,5 und 26,0 At.-% liegt.

2. Verwendung der bioresorbierbaren Metalllegierungszusammensetzung in einem vaskulären medizinischen Implantat nach Anspruch 1, wobei der Fe-Gehalt zwischen 43,0 und 50,5 At.-% liegt.

3. Verwendung der bioresorbierbaren Metalllegierungszusammensetzung in einem vaskulären medizinischen Implantat nach einem der vorhergehenden Ansprüche, wobei der Mn-Gehalt zwischen 34,0 und 37,5 At.-% liegt.

4. Verwendung der bioresorbierbaren Metalllegierungszusammensetzung in einem vaskulären medizinischen Implantat nach einem der vorhergehenden Ansprüche, wobei der X-Element-Gehalt zwischen 10,0 und 15,0 At.-% liegt.

5. Verwendung der bioresorbierbaren Metalllegierungszusammensetzung in einem vaskulären medizinischen Implantat nach einem der vorhergehenden Ansprüche, wobei der Y-Element-Gehalt zwischen 5,0 und 8,0 At.-% liegt.

6. Verwendung der bioresorbierbaren Metalllegierungszusammensetzung in einem vaskulären medizinischen Implantat nach einem der vorhergehenden Ansprüche, wobei die Summe von X und Y zwischen 13,0 und 25,0 At.-% liegt.

7. Verwendung der bioresorbierbaren Metalllegierungszusammensetzung in einem vaskulären medizinischen Implantat nach einem der vorhergehenden Ansprüche, wobei das Verhältnis von X und Y zwischen 1,5 und 3,2 liegt.

8. Verwendung der bioresorbierbaren Metalllegierungszusammensetzung in einem vaskulären medizinischen Implantat nach Anspruch 1, wobei das X-Element Aluminium ist.

9. Verwendung der bioresorbierbaren Metalllegierungszusammensetzung in einem vaskulären medizinischen Implantat nach einem der Ansprüche 1 bis 7, wobei das X-Element Zink ist.

10. Verwendung der bioresorbierbaren Metalllegierungszusammensetzung in einem vaskulären medizinischen Implantat nach einem der vorhergehenden Ansprüche, wobei das vaskuläre medizinische Implantat ausgewählt ist aus einer Gruppe bestehend aus peripheren vaskulären Stents, koronaren vaskulären Stents, neurovaskulären Stents, Trachealstents, Gallengangstents und Ösophagusstents oder Implantaten in Hohlräumen und Trakten.

11. Ein bioresorbierbares vaskuläres medizinisches Implantat umfassend eine bioresorbierbare Metalllegierungszusammensetzung bestehend aus Fe-Mn-X-Y plus unvermeidbaren Verunreinigungen, die 0,5 At.-% nicht übersteigen, und wobei der Fe-Gehalt höher ist als der Mn-Gehalt, der Fe-Gehalt in einer Menge zwischen 35,0 und 50,5 At.-% liegt, der Mn-Gehalt in einer Menge zwischen 34,0 und 40,0 At.-% liegt, X mindestens ein Element ausgewählt aus der Gruppe bestehend aus Aluminium, Zink, Gallium und Kombinationen davon in einer Menge zwischen 8,0 und 16,0 At.-% ist, und Y Kupfer oder Kohlenstoff oder eine Kombination davon in einer Menge zwischen 4,5 und 10,0 At.-% ist, wobei das atomare Verhältnis zwischen X und Y zwischen 1,0 und 3,6 liegt, und die Summe von X und Y zwischen 12,5 und 26,0 At.-% liegt.

12. Ein bioresorbierbares vaskuläres medizinisches Implantat nach Anspruch 11, wobei der Fe-Gehalt in einer Menge zwischen 43,0 und 50,5 At.-% liegt, der Mn-Gehalt in einer Menge zwischen 34,0 und 37,5 At.-% liegt, X in einer Menge zwischen 10,0 und 15,0 At.-% liegt und Y in einer Menge zwischen 5,0 und 8,0 At.-% liegt, wobei das atomare Verhältnis zwischen X und Y zwischen 1,5 und 3,2 liegt und die Summe von X und Y zwischen 13,0 und 25,0 At.-% liegt.

13. Ein bioresorbierbares vaskuläres medizinisches Implantat nach Anspruch 11 oder 12, ausgewählt aus einer Gruppe bestehend aus peripheren vaskulären Stents, koronaren vaskulären Stents, neurovaskulären Stents, Trachealstents, Gallengangstents und Ösophagusstents oder Implantaten in Hohlräumen und Trakten.

14. Ein bioresorbierbares vaskuläres medizinisches Implantat nach Anspruch 11 oder 12, wobei das medizinische Implantat ein dünnwandiges medizinisches Implantat ist.

## Revendications

1. Utilisation, dans un implant médical vasculaire, d'une composition d'alliage métallique biorésorbable consistant en Fe-Mn-X-Y plus des impuretés inévitables qui ne dépassent pas 0,5 % atomiques, dans laquelle la quantité de Fe est supérieure à la quantité de Mn, **caractérisée en ce que** la teneur en Fe est une quantité comprise entre 35,0 et 50,5 % atomiques, la teneur en Mn est une quantité comprise entre 34,0 et 40,0 % atomiques, X est au moins un élément choisi dans le groupe constitué par l'aluminium, le zinc, le gallium et leurs combinaisons, en une quantité comprise entre 8,0 et 16,0 % atomiques, Y est le cuivre ou le carbone ou une combinaison de ceux-ci en une quantité comprise entre 4,5 et 10,0 % atomiques, le rapport atomique entre X et Y est compris entre 1,0 et 3,6, et la somme de X et Y est comprise entre 12,5 et 26,0 % atomiques.

2. Utilisation, dans un implant médical vasculaire, de la composition d'alliage métallique biorésorbable selon la revendication 1, dans laquelle la teneur en Fe est comprise entre 43,0 et 50,5 % atomiques.

3. Utilisation, dans un implant médical vasculaire, de la composition d'alliage métallique biorésorbable selon l'une quelconque des revendications précédentes, dans laquelle la teneur en Mn est comprise entre 34,0 et 37,5 % atomiques.

4. Utilisation, dans un implant médical vasculaire, de la composition d'alliage métallique biorésorbable selon l'une quelconque des revendications précédentes, dans laquelle la teneur en l'élément X est comprise entre 10,0 et 15,0 % atomiques.

5. Utilisation, dans un implant médical vasculaire, de la composition d'alliage métallique biorésorbable selon l'une quelconque des revendications précédentes, dans laquelle la teneur en l'élément Y est comprise entre 5,0 et 8,0 % atomiques.

6. Utilisation, dans un implant médical vasculaire, de la composition d'alliage métallique biorésorbable selon l'une quelconque des revendications précédentes, dans laquelle la somme de X et Y est comprise entre 13,0 et 25,0 % atomiques.

7. Utilisation, dans un implant médical vasculaire, de la composition d'alliage métallique biorésorbable selon l'une quelconque des revendications précédentes, dans laquelle le rapport de X à Y est compris entre 1,5 et 3,2.

8. Utilisation, dans un implant médical vasculaire, de la composition d'alliage métallique biorésorbable selon la revendication 1, dans laquelle l'élément X est l'aluminium.

9. Utilisation, dans un implant médical vasculaire, de la composition d'alliage métallique biorésorbable selon l'une quelconque des revendications 1 à 7, dans laquelle l'élément X est le zinc.

10. Utilisation, dans un implant médical vasculaire, de la composition d'alliage métallique biorésorbable selon l'une quelconque des revendications précédentes, dans laquelle l'implant médical vasculaire est choisi dans le groupe constitué par les stents vasculaires périphériques, les stents vasculaires coronariens, les stents neurovasculaires, les stents trachéaux, les stents du canal cholédoque et les stents oesophagiens ou les implants dans les cavités et tractus.

11. Implant médical vasculaire biorésorbable comprenant une composition d'alliage métallique biorésorbable consistant en Fe-Mn-X-Y plus des impuretés inévitables qui ne dépassent pas 0,5 % atomiques, dans lequel la quantité de Fe est supérieure à la quantité de Mn, la teneur en Fe est une quantité comprise entre 35,0 et 50,5 % atomiques, la teneur en Mn est une quantité comprise entre 34,0 et 40,0 % atomiques, X est au moins un élément choisi dans le groupe constitué par l'aluminium, le zinc, le gallium et leurs combinaisons, en une quantité comprise entre 8,0 et 16,0 % atomiques, Y est le cuivre ou le carbone ou une combinaison de ceux-ci en une quantité comprise entre 4,5 et 10,0 % atomiques, dans lequel le rapport atomique entre X et Y est compris entre 1,0 et 3,6, et la somme de X et Y est comprise entre 12,5 et 26,0 % atomiques.

12. Implant médical vasculaire biorésorbable selon la revendication 11, dans lequel la teneur en Fe est comprise entre 43,0 et 50,5 % atomiques, la teneur en Mn est comprise entre 34,0 et 37,5 % atomiques, X est présent en une quantité comprise entre 10,0 et 15,0 % atomiques et Y est présent en une quantité comprise entre 5,0 et 8,0 % atomiques, dans lequel le rapport atomique entre X et Y est compris entre 1,5 et 3,2 et la somme de X et Y est comprise entre 13,0 et 25,0 % atomiques.

13. Implant médical vasculaire biorésorbable selon la revendication 11 ou 12, choisi dans le groupe constitué par les stents vasculaires périphériques, les stents vasculaires coronariens, les stents neurovasculaires, les stents trachéaux, les stents du canal cholédoque et les stents oesophagiens ou les implants dans les cavités et tractus.

14. Implant médical vasculaire biorésorbable selon la revendication 11 ou 12, lequel implant médical est un implant médical à paroi mince.
